# EUROPÄISCHE PATENTSCHRIFT

(11) **EP 0 811 359 B1**
(45) Veröffentlichungstag und Bekanntmachung des Hinweises auf die Patenterteilung: **10.07.2002**
(21) Anmeldenummer: 97108068.4
(22) Anmeldetag: 21.10.1991
(51) Int. Cl.: A61F 2/28, A61L 27/00, C04B 38/04, C04B 38/06, C08J 9/26, C22C 1/00

(54) **Verfahren zur Herstellung eines Werkstoffs**
Process for manufacturing of a material
Procédé de matériau

(30) Priorität: 19.10.1990 DE 4033291
(43) Veröffentlichungstag der Anmeldung: 10.12.1997
(62) Teilanmeldung aus: 91918051.3
(73) Patentinhaber: Draenert, Klaus, Dr.med.Dr.med.habil., D-81545 München (DE)
(72) Erfinder: Draenert, Klaus, Dr.med.Dr.med.habil., D-81545 München (DE)

(56) Entgegenhaltungen:
- EP-A- 0 107 476
- EP-A- 0 277 678
- DE-A- 2 242 867
- GB-A- 2 093 701
- US-A- 4 859 712
- US-A- 4 891 182

## Beschreibung

Die Erfindung betrifft ein Verfahren zu Herstellung eines Werkstoffes gemäß Anspruch 1.

Es besteht allgemein und insbesondere in der Medizin ein Bedürfnis nach Werkstoffen, die unter größtmöglicher Materialeinsparung und/oder bei niedrigem Gewicht ein Höchstmaß an Festigkeit bieten. Es besteht auch ein Bedürfnis, beispielsweise in der Filtertechnologie, nach Werkstoffen, die eine Struktur aufweisen, mit der es bei durchgehender Porosität des Werkstoffes gelingt, entlang großer Oberflächen Gase oder Flüssigkeiten zu absorbieren bzw. Teilchen in einem einstellbaren Hohlraumsystem des Werkstoffes festzuhalten.

Aus der DE-C2-31 06 917 ist ein Verfahren zur Herstellung eines Implantates als Knochenersatz in Form eines offenporigen bzw. offenzelligen Formkörpers aus körperverträglichem Metall bekannt, wobei das Metall unter Anwendung eines verlorenen Modells verarbeitet wird. Bei diesem Verfahren werden als Positivmodell offenporige bzw. offenzellige Naturoder Kunstschwämme mit einer durchschnittlichen Weite der Poren oder Zellen zwischen 0,5 und 1,5 mm verwendet, die mit einer keramischen Einbettmasse gefüllt werden. Anschließend wird das Modellmaterial durch Hitze zerstört und entfernt, wodurch ein Keramik-Negativmodell entsteht. Danach werden die zuvor von dem Material des Positivmodells, d.h. dem Natur- oder Kunstschwamm, eingenommenen Räume durch ein gießoder schleuderbares Metall gefüllt und anschließend wird das Keramikmaterial des Negativmodells wieder entfernt. Ein Nachteil dieses Verfahrens besteht darin, daß die Struktur des Positivmodells und damit die Struktur des fertigen Metallimplantates nicht gezielt einstellbar ist, sondern daß die Struktur jeweils so akzeptiert werden muß, wie die Schwämme, seien es Natur- oder Kunstschwämme, beschaffen sind. Ein weiterer Nachteil dieses Verfahrens besteht darin, daß es umständlich ist und zwei Formkörper, d.h. zunächst ein Positivmodell und danach ein Negativmodell, benötigt werden, um letztlich ein dreidimensionales Gerüst zu schaffen.

In der DE-A-29 10 627 wird vorgeschlagen, ein aus Fäden oder Fasern aus Kunststoff oder Metall aufgebautes formstabiles Netzwerk vorzugeben und dieses als Implantat zu verwenden. Dieser Vorschlag ist aber schwer realisierbar, da die Netzwerke sehr schwierig und teuer herzustellen sind und es kaum gelingt, die Formstabilität reproduzierbar sicherzustellen und eine entsprechende Tragfähigkeit des Implantats zu garantieren. Dieses Patent lehrt aus einer Positivstruktur wieder eine Positivstruktur zu erhalten. Das erfindunsgemäße Verfahren lehrt aus einem Negativ wieder eine Negativstruktur aus einem anderen Material zu erhalten.

Der Erfindung liegt somit die Aufgabe zugrunde, ein Verfahren zur Herstellung eines Werkstoffs bereitzustellen, das es ermöglicht, ein dreidimensionales Gerüst sowohl im Hinblick auf die Porosität des Werkstoffes und die Dicke der tragenden Strukturen als auch im Hinblick auf die Formstabilität und andere gewünschte Eigenschaften, wie Löslichkeit oder Resorbierbarkeit, exakt nach den jeweiligen Bedürfnissen einzustellen.

In der Offenlegungsschrift 2 242 867 wird ein Verfahren beschrieben, welches uber Negativformkörper ein "Positiv-"gerürt erzeugt, jedoch keinen Weg aufzeigt zu einem "Negativ" implantat.

Diese Aufgabe wird durch die vorliegende Erfindung gemäß den Patentansprüchen gelöst. Die Erfindung löst somit ein altes Problem in der Werkstoffherstellung, das insbesondere für die Medizin sehr große Bedeutung hat, nämlich die Herstellung von durchgehend porösen Gerüsten mit einer einstellbaren Porosität und einer einstellbaren Festigkeit sowie jeweils den Bedürfnissen angepaßten Materialeigenschaften, wie Löslichkeit und Resorbierbarkeit. Dabei wird vorzugsweise jeweils von einem Negativmodell des fertigen Werkstoffs ausgegangen.

Die Erfindung geht von dem Grundgedanken aus, miteinander verbundene einzelne Formkörper, z.B. in Form einer dichten Packung oder eines Konglomerats, als Platzhalter für die Hohlraumstruktur zu verwenden und um die leicht entfernbaren, beispielsweise leicht löslichen oder leicht schmelzbaren Förmkörper herum ein dreidimensionales Gerüst aus tragenden Strukturen zu schaffen, vorzugsweise aus einem gießfähigen Material. Im Anschluß daran können die Formkörper entweder physikalisch oder chemisch, vorzugsweise durch Anwendung des Prinzips der Wasserlöslichkeit oder der Schmelzbarkeit, oder durch Anwendung von Hitze wieder entfernt werden. Erfindungsgemäß können auch die einzelnen Formkörper selbst im Sinne eines Positiv-/Negativmodells hergestellt werden.

Anders ausgedrückt beruht die Erfindung im Prinzip darauf, daß durch Formkörper vorgegebener Konfiguration, beispielsweise kugelförmige oder granulare Formkörper, welche punktförmig miteinander zu einer dreidimensionalen Struktur in Form eines Konglomerats verbünden werden, eine durchgehende bälkchenförmige oder trabekuläre Hohlraumstruktur entsteht; diese Hohlraumstruktur wird anschließend mit einem anderen Material ausgefüllt. Die räumliche Struktur des zum Ausfüllen verwendeten Materials stellt das "Positivmodell" zu dem von dem Formkörper-Konglomerat gebildeten "Negativmodell" dar und ist durch geeignete Wahl der Struktur des Negativmodells beliebig einstellbar. Der nach dem Ausfüllen entstandene Verbundkörper kann anschließend von den ein "Inlet" bildenden Formkörpern befreit werden. Durch die Entfernung der Formkörper entsteht ein durchgehend poröser Werkstoff. Die Porosität des Werkstoffes ist durch geeignete Auswahl der Größe, Größenverteilung und Schüttdichte der Formkörper sowie der Art deren gegenseitiger Verbindung reproduzierbar einstellbar. Der fertige Werkstoff ist mechanisch widerstandsfähig und kann in seiner äußeren Form beliebig gestaltet werden.

Der Werkstoff unterscheidet sich von mit herkömmlichen Verfahren hergestellten Werkstoffen, beispielsweise aus der Zahntechnik dadurch, daß sich sowohl die Porosität als auch die Stärke der trabekelartigen tragenden Strukturen und die Festigkeit exakt einstellen lassen.

Der Werkstoff findet insbesondere in der Medizintechnik und dort ganz besonders für die Herstellung von Implantaten oder auch als Wirkstoffträger in sogenannten "drug delivery"-Systemen Verwendung. Der Werkstoff kann aber auch als Träger für alle diejenigen Wirkstoffe verwendet werden, die auf Oberflächen aufgetragen werden können und zu denen beispielsweise das "bone morphogenetic protein" oder gewisse Wachstumsfaktoren zählen.

Entlang der vorzugsweise beschichteten Oberflächen der vorgebbar einstellbaren Hohlraumstruktur kommt es erwiesenermaßen sehr schnell zu einem gezielten Knocheneinwuchs, der bei Verwendung des erfindungsgemäßen Werkstoffs als Implantat das gesamte Implantat auch unabhängig von der biomechanischen Beanspruchung knöchern durchwachsen kann.

Die Werkstoffe sind auch in der Filtertechnologie verwendbar, da jede beliebige Porosität einstellbar ist und jedes beliebige aktiv absorbierende Material in Kombination mit einer beliebigen Porosität verwendet werden kann. Mit dem erfindungsgemäßen Werkstoff ist ein Filter herstellbar, bei dem ein makroskopisch großporiges Filtersystem mit einem mikroporösen Filtersystem kombiniert werden kann.

Im Prinzip können alle beliebigen Werkstoffe in die Negativoder Positivform gebracht werden, indem beispielsweise wasserlösliche oder säurelösliche Formkörper mit schmelzbaren Materialien oder wasserlösliche oder säurelösliche Formkörper mit sinterfähigen, gießbaren Materialverbunden oder im Spritzgußverfahren verarbeitbaren Kunststoffen oder gießbaren, beispielsweise im Schleuderguß oder Spritzguß verarbeitbaren Metallen, Metallegierungen oder Metallverbundwerkstoffen in der Weise kombiniert werden, daß jeweils eine Sorte Formkörper, entweder physikalisch oder chemisch oder auf andere Weise, wieder herauslösbar ist und das dreidimensionale Gerüst als Stützgerüst verbleibt. Das Stützgerüst kann anschließend durch physikalische oder chemische Verfahren verfestigt, oberflächenbehandelt oder mechanisch nachbearbeitet werden.

Die Formkörper und das vorzugsweise aus einem gießbaren Material bestehende Stützgerüst können auch zusammen, beispielsweise mechanisch, weiterverarbeitet werden und erst im Anschluß daran physikalisch oder chemisch separiert werden. Die so erhaltenen dreidimensionalen Gerüste können wiederum temporär als Formkörper für Negativmodelle dienen, so daß sowohl das dreidimensionale Gerüst mit durchgehender Porosität als auch. die Packung bzw. das Konglomerat aus miteinander verbundenen und vorzugsweise kugelförmigen Formkörpern wechselseitig als Modell eingesetzt werden können. Auf diese Weise können alle möglichen Materialien, Materialkombinationen und Verbundwerkstoffe zu dem erfindungsgemäßen Werkstoff verarbeitet werden.

Das Material für das dreidimensionale Gerüst ist vorzugsweise gießbar oder spritzbar, beispielsweise im Spritzgußverfahren. Falls der Werkstoff als Implantat verwendet wird, besteht das dreidimensionale Gerüst des erfindungsgemäßen Werkstoffes vorzugsweise aus einem Polymer auf der Basis eines Polyacrylates oder eines Polymethacrylates, einem Copolymer eines Acrylates und Methacrylates, einer Mischung aus diesen oder aus einem anderen körperverträglichen Kunststoff. Das dreidimensionale Gerüst kann auch aus einer resorbierbaren Polyaminosäure, einem Polylactat, einem Polyglykolat, einem Gemisch aus verschiedenen Polyaminosäuren oder einem anderen im Körper auflösbaren und/oder resorbierbaren Material bestehen. Silikon, ein Kautschukderivat oder ein verwandtes Polymer auf Gummibasis ist als Material für das dreidimensionale Gerüst bevorzugt, falls der Werkstoff als Filter, vorzugsweise als Nasenfilter verwendet wird.

Die als Platzhalter für das Hohlraumsystem des Werkstoffes dienenden Formkörper weisen vorzugsweise die Form von Kugeln oder gleichmäßigen geometrischen Körpern, beispielsweise Vielecken auf, es kann aber auch Granulatmaterial als Formkörper verwendet werden. Vorzugsweise ist das Material der Formkörper leicht löslich, z.B. wasserlöslich oder säurelöslich, oder leicht schmelzbar. Besonders bevorzugt sind Formkörper aus einem wasserlöslichen Material, beispielsweise aus Zucker, die zunächst im Wasserdampf zur Bildung eines Formkörperkonglomerats fest miteinander verklebt werden können und nach der Ausbildung und Aushärtung des dreidimensionalen Gerüsts ausgewaschen werden können, beispielsweise im Wasserbad oder in einer Waschmaschine.

Als Material für die Formkörper kann ein anorganischer oder keramischer Werkstoff, beispielsweise Tricalciumphosphat, ein Hydroxylapatit, ein Gemisch aus beiden oder eine andere, leicht oder schwer resorbierbare Calciumverbindung verwendet werden, insbesondere wenn der Werkstoff für ein Implantat verwendet wird. Bevorzugt sind säurelösliche keramische Werkstoffe.

Da sowohl die Größe als auch die Schüttdichte und die Art der gegenseitigen Verbindung der als Platzhalter für das Hohlraumsystem des Werkstoffes dienenden Formkörper frei wählbar ist, ist auch das Hohlraumsystem des fertigen Werkstoffes bezüglich seiner Porosität frei einstellbar. Beispielsweise können kugelförmige Formkörper verwendet werden, wenn ein Hohlraumsystem mit im wesentlichen kugelförmigen und miteinander in Verbindung stehenden Hohlräumen angestrebt wird. Wenn ein Hohlraumsystem mit unterschiedlich großen Hohlräumen angestrebt wird, können Formkörper unterschiedlicher Größe und/oder Form miteinander gemischt werden. Die gesamte Porosität des fertigen Werkstoffes ist durch Einstellung, Variation und Kombination der Form und/oder der Schüttdichte der Formkörper und/oder Wahl des Verfahrens, durch das die Formkörper zu einem Konglomerat miteinander verbunden werden, gezielt einstellbar. Die Form und Gestaltung der Bälkchen bzw. Trabekel des dreidimensionalen Gerüsts ist erfindungsgemäß ebenso vorgebbar und gezielt je nach Verwendungszweck einstellbar.

Die Formkörper können beispielsweise durch ein Sinterverfahren miteinander verbunden werden. Die bevorzugte Größe der Formkörper beträgt zwischen etwa 0,5 und 5 mm, besonders bevorzugt bis 3 mm, insbesondere falls der Werkstoff als Implantat oder als Filter verwendet wird.

Der Werkstoff kann auch zusätzlich Füllerpartikel enthalten, beispielsweise Tricalciumphosphat oder Hydroxylapatit oder ein Gemisch aus beiden mit einem Anteil von 1 bis 95 %, vorzugsweise 1 bis 80 %, und mit einer Partikelgröße von 50 bis 300 µm, bis zu 250 µm, und einem Porenvolumen von 0,1 ml/g bis 0,8 ml/g.

Je nach der beabsichtigten Verwendung können dem Werkstoff auch verschiedene Wirkstoffe zugesetzt werden, beispielsweise kann das Material für das dreidimensionale Gerüst 0,01 bis 10 % eines Wirkstoffes enthalten, der aus dem Werkstoff verzögert freigesetzt werden kann. Als Wirkstoffe kommen bei Verwendung des Werkstoffes als Implantat beispielsweise Antibiotika, ein das Knochenwachstum induzierender Wirkstoff, ein Wachstumsfaktor oder ein anderer chemotaktisch oder hormonell wirkender Faktor in Betracht, der das Einsprossen der Gefäße oder direkt die Stimulation der Osteoblasten bewirkt. Falls der Werkstoff als Filter verwendet wird, kann das dreidimensionale Gerüst mit Aktivkohle oder einem anderen, stark gas- und/oder flüssigkeitsabsorbierenden Füller versetzt werden, beispielsweise in einer Konzentration zwischen 5 und 80 Gew.-%.

Die äußere Form des Werkstoffes ist frei wählbar und richtet sich nach dem jeweiligen Verwendungszweck.

Die Beispiele 1-5 erläuten nur den Hintergrund fur das erfindungsgemäße Verfahren.

### Beispiele

### Beispiel 1

### Herstellung eines porösen Implantates mit hoher mechanischer Festigkeit

Keramikkugeln mit einem Durchmesser zwischen 0,5 und 3 mm werden in einem Sinterofen zusammengesintert und punktförmig zu einem Konglomerat in Form eines porösen Körpers verschweißt, der als Negativmodell dient. Die miteinander in Verbindung stehenden bzw. interkonnektierenden Poren (Hohlräume) des Konglomerats werden mit einem gießförmigen Metall im Schleudergußverfahren ausgefüllt. Der so entstandene Verbundwerkstoff aus Keramik und Metall wird anschließend im sogenannten "HIP-Verfahren" sehr hoch verdichtet. Danach wird die Keramik auf chemischem Wege, beispielsweise durch Anwendung von Säure, wieder entfernt, und es verbleibt ein Werkstoff aus einem dreidimensionalen Metallgerüst mit tragenden bälkchenartigen Strukturen und einem interkonnektierenden Porensystem, das beispielsweise als Implantat verwendbar ist.

### Beispiel 2

### Herstellung eines hochfesten keramischen Implantatkörpers

Metallkugeln einer Größe zwischen 0,5 und 3 mm werden als Formkörper verwendet und durch Hitze oder punktförmige Verklebung miteinander verschweißt bzw. verbunden, so daß ein durchgehendes trabekuläres System von Hohlräumen um die Kugeln entsteht. Diese sehr festen Formkörper werden anschließend mit einer keramischen Masse im Schleudergußverfahren umgossen. Danach wird der entstandene Verbundwerkstoff im "HIP-Verfahren" hoch verdichtet. Anschließend werden die Metallkugeln auf elektrolytischem Wege herausgelöst. Das verbleibende dreidimensionale Keramikgerüst wird im Sinterverfahren weiter verfestigt. Auf diese Weise wird ein hochfester keramischer Implantatkörper mit dreidimensionaler trabekulärer Struktur hergestellt. Die Poren des durchgehenden Hohlraumsystems sind durch geeignete Wahl der Größe der als Platzhalter dienenden Metallkugeln einstellbar.

### Beispiel 3

### Herstellung eines leistungsfähigen Filters

Zuckerkugeln einer Größe zwischen 0,5 und 3 mm werden lose geschüttet und im Wasserdampf kurzfristig miteinander unter Anwendung von Vakuum verklebt. Anschließend wird das Konglomerat getrocknet und mit einem gießfähigen Silikongemisch, welches mit bis zu 80 % Aktivkohle versetzt sein kann, ausgegossen. Nach Aushärtung des Silikons wird der Zucker anschließend in Wasser, beispielsweise in einer Waschmaschine, wieder entfernt. Es wird ein dreidimensionaler Filterkörper aus Silikon aus einem trabekulären Gerüst mit einer hohen aktiv absorbierenden Oberfläche erhalten. Das Porensystem des Filterkörpers ist durch geeignete Wahl der Größe und Größenverteilung der Zuckerkugeln einstellbar. Das Filter kann beispielsweise als Nasenfilter in das Vestibulum nasi sehr wirksam zur Verhütung der Verschmutzung der Atemwege eingesetzt werden. Vorzugsweise wird die Form des Filters an die Form des Vestibulum nasi angepaßt.

### Beispiel 4

### Herstellung eines Verbundwerkstoffes

Zuckerkugeln einer Größe von 0,5 bis 3 mm werden in loser Schüttung in eine Form gegeben und anschließend unter Vakuumanwendung von Wasserdampf durchströmt und miteinander verklebt. Nach einer anschließenden Trocknung wird die so entstandene Masse (Konglomerat) mit einem flüssigen Knochenzement auf PMMA-Basis ausgegossen oder im Spritzgußverfahren ausgespritzt. Nach Aushärtung des Knochenzements bzw. des thermisch geschmolzenen Kunststoffes wird der Zucker ausgewaschen, beispielsweise in einer Waschmaschine. Der fertige Werkstoff in Form eines porösen Körpers hat eine gleichförmige dreidimensionale Bälkchenstruktur und kann in Form eines Zylinders als zentrales Markraumimplantat eingebracht werden. Dieses Implantat kann Knochenschrauben in einem osteoporotischen, biomechanisch sehr stark geschwächten Knochen einen festen Halt bieten und kann so, beispielsweise bei mehreren Fragmenten und Epiphysenfrakturen zu einer anatomisch exakten Reposition führen. Das unter Verwendung des erfindungsgemäßen Werkstoffes herstellbare Implantat ist auch mechanisch bearbeitbar und kann vollständig von Knochen durchwachsen werden. Wie vorstehend erläutert, ist der Werkstoff beispielsweise zur Verankerung von Knochenschrauben bei sogenannten "Verbundosteosynthesen" oder als Plug, d.h. als sogenannter Markraumsperrer bei zementierten Prothesenkomponenten einsetzbar.

### Beispiel 5

### Herstellung eines als Prothesenschaft für eine Femurkomponente einer Hüftgelenksersatzprothese verwendbaren Implantatkörpers

In eine anatomisch geformte erste Form werden Keramikkugeln einer Größe zwischen 0,5 und 1,5 mm lose aufgeschüttet und im Sinterofen zusammengesintert. Der erhaltene Körper (Konglomerat) aus gesintertem Material wird anschließend in einer ähnlich aufgebauten zweiten Form, die jedoch schalenförmig etwa 3 bis 4 mm weiter ist als die erste Form, so versenkt, daß ein gleichförmiger Spalt um den gesinterten Körper verbleibt. Dieser Spalt wird mit Keramikkugeln einer Größe von 1 bis 3 mm aufgefüllt. Anschließend wird die gefüllte zweite Form in einem Sinterofen gesintert, so daß aus den beiden unterschiedlichen Kugelkomponenten ein Verbundkörper aus gesinterten Kugeln entsteht. Dieser Verbundkörper wird als Modell im Schleudergußverfahren mit einer gießbaren Metallegierung ausgegossen und anschließend im "HIP-Verfahren" so verdichtet, daß ein hochfester Metallkeramikverbundkörper entsteht. Im Anschluß daran wird die Keramik zum größten Teil bis auf eine Oberflächenbeschichtung des Metalles wieder entfernt, vorzugsweise auf chemischem Wege. Auf diese Weise entsteht ein hochfester, vollständig poröser Metallkörper, der im Bereich seiner Oberfläche unterschiedliche mechanische Eigenschaften gegenüber dem massiveren Innenteil mit dichterer Struktur aufweist. Diese Oberfläche stellt bei Verwendung des Metallkörpers als Implantat oder Prothese die Grenzzone zum Knochen dar. Eine solche Schichtung kann auch in axialer Richtung in verschiedener Weise wiederholt eingestellt werden, so daß insgesamt jede beliebige Prothesenschaft-Konfiguration mechanisch einstellbar und reproduzierbar gefertigt werden kann. Die optimale Prothesenschaft-Konfiguration kann beispielsweise nach Finite-Elemente-Methoden errechnet werden. Es lassen sich somit auch sehr kompliziert aufgebaute Implantatkörper oder Prothesen mit einem durchgehenden inneren Hohlraumsystem beliebig und gezielt herstellen.

## Patentansprüche

1. Verfahren zum Herstellen eines Werkstoffes mit den folgenden Verfahrensschritten:
Bildung einer dreidimensionalen Packung oder eines Konglomerates von miteinander in Verbindung stehenden löslichen oder schmelzbaren Formkörpern,
Formen eines dreidimensionalen Gerüsts aus einem sich vom Material der Formkörper unterscheidenden, Wasserlöslichen oder schmelzbaren Materials um die Formkörper herum ,
Aushärten des Gerüsts, Herauslösen oder Entfernen des Formkörpermaterials durch Anwendung von Hitze,
Füllen des durch Entfernen der Formkörper entstandenen durchgehenden Hohlraumsystems mit Porengrössen zwischen 0,2 und 5mm, vorzugsweise 3mm, mit einer Keramikmasse, und
Entfernen des Gerüsts aus dem löslichen oder Schmelzbaren Material durch Anwendung von Wasser oder Hitze .

2. Verfahren nach Anspruch 01, wobei die Formkörper als eine variierbare dichte Kugelpakkung einzelner Kugeln oder als Kugelkonglomerat aus einzelnen fest miteinander verbundenen Kugeln vorliegen.

3. Verfahren nach den Ansprüchen 01 bis 02,wobei die Formkörper aus gleichmässigen geometrischen Körpern,Vielecken,Granulat oder aus einem Gemisch aus verschiedenen gleichförmigen oder ungleichförmigen Formkörpern bestehen und zwar in ausgewählt dichter Schüttung oder aus einem Konglomerat aus fest miteinander verbundenen Formkörpern.

## Claims

1. Process for manufacturing a material comprising the following steps:
formation of a three-dimensional package or a conglomerate of soluble shaped bodies or shaped bodies capable of melting which are connected to each other,
formation of a three-dimensional framework around the shaped bodies from a material which is different from the material of the shaped bodies and which is soluble in water or is capable of melting,
hardening of said framework, dissolving out or removal of the shaped body material with the use of heat,
filling of the continuous hollow space system created by removal of the shaped body material with pore sizes between 0.2 mm and 5 mm, preferably 3 mm, with a ceramic mass and
removing of the framework made from the soluble material or material capable of melting with the use of water or heat.

2. Process according to Claim 1, wherein the shaped bodies are in the form of a ball package or variable density made up of individual balls or in the form of a ball conglomerate of individual balls connected with each other in a fixed fashion.

3. Process according to Claims 1 und 2, wherein the shaped bodies consist of symmetrical bodies, polygons, granulate or a mixture of uniform or non-uniform shaped bodies in a bulk whose density can be selected or of a conglomerate of shaped bodies which are connected to one another in a fixed fashion.

## Revendications

1. Procédé de fabrication d'un matériau selon les étapes de fabrication suivantes:
Réalisation d'un enveloppement tridimensionel ou d'un conglomérat de corps moulants reliés entre eux, résorbables ou pouvant fondre.
Configuration d'une charpente tridimensionelle réalisée en un matériau qui se distingue de celui des corps moulants, résorbable ou pouvant fondre, entourant les corps moulants.
Durcissement de la charpente, extraction ou élimination du matériau des corps moulants par application de la chaleur.
Remplissage du système de cavités généré de part en part à la suite de l'enlèvement des corps moulants, avec une grosseur des pores entre 0,7 et 5 mm, de préférence 3 mm, au moyen d'une masse céramique et
Enlèvement de la charpente à partir du matériau résorbable ou pouvant fondre en utilisant de l'eau ou de la chaleur.

2. Procédé selon revendication 1, les corps moulants se présentant en tant qu' enveloppement en forme de boule de densité variable comportant des boules isolées ou en tant que conglomérat en forme de boules comportant des boules isolées solidement réunies entre elles.

3. Procédé selon les revendications 1 et 2, les corps moulants étant constitués par des corps géométriques de masse uniforme, par des polygones, des granulés ou par un mélange de divers corps moulants uniformes ou non uniformes et, cela, en tas de densité choisie ou en conglomérat de corps moulants solidement réunis.
